# EUROPEAN PATENT SPECIFICATION

(11) **EP 0 257 967 B1**
(45) Date of publication and mention of the grant of the patent: **19.05.1993**
(21) Application number: 87307313.4
(22) Date of filing: 19.08.1987
(51) Int. Cl.: C07C 29/15, C07C 31/20, C07C 31/27

(54) **Process for making 1,3-glycols from epoxides**
Herstellungsverfahren von 1,3-Glykolen aus Epoxyden
Procédé de fabrication de 1,3 glycols à partir d'époxydes

(30) Priority: 20.08.1986 US 898072
(43) Date of publication of application: 02.03.1988
(73) Proprietor: HOECHST CELANESE CORPORATION, Somerville, N.J. 08876 (US)
(72) Inventor: Murphy, Mark Alan, Corpus Christi Texas (US); Aguilo, Adolfo, Corpus Christi Texas (US); Smith, Brad Lee, Portland Texas (US)
(74) Representative: De Minvielle-Devaux, Ian Benedict Peter

(56) References cited:
- EP-A- 0 034 374
- US-A- 3 463 819

## Description

This invention relates to the manufacture of 1,3-diols, in general, and in particular to the manufacture of 1,3-diols from an epoxide. In one embodiment, this invention relates to the manufacture of 1,3-propanediol from ethylene oxide.

Glycols in general are valuable chemical compounds which find a wide variety of utilities. Such compounds are used, for example, as chemical intermediates in the manufacture of esters, as well as in the synthesis of polyesters. 1,3-propanediol, also referred to as 1,3-propylene glycol or trimethyleneglycol, in particular, has been found to be especially useful in a number of applications. Typically, 1,3-propanediol has been prepared by acid-catalyzed hydration of acrolein to form hydracrylaldehyde which is subsequently hydrogenated to the corresponding glycol. The high cost of acrolein and the relatively low yields obtained in such reactions have not led to commercial processes for production of 1,3-propanediol which are cost competitive with other commercially available diols which in many instances can be substituted for 1,3-propanediol.

The preparation of 1,3-glycols by the hydroformylation of epoxides, utilizing phosphine-modified cobalt carbonyl complexes as the catalyst, is shown in U.S. Patent No. 3,463,819. In particular, said patent shows the production of 1,3-propanediol by hydroformylation of ethylene oxide, using a tertiary phosphine-modified cobalt carbonyl component per mol of ethylene oxide. Although high yields (92% of 1,3-propanediol were claimed to have been produced in diethyl ether solvent, catalyst concentrations were extremely high, the amount of ethylene oxide charged was low, and no indication of reaction times nor reaction rates was specified. Yields of 1,3-propanediol were substantially lower in solvents other than diethyl ether.

U.S. Patent No. 3,687,981 is also directed to a process for manufacturing 1,3-propanediol. However, the process disclosed in the ʹ981 patent employs two separate stages. In the first stage ethylene oxide undergoes a hydroformylation reaction to produce hydroxyethyl hydroxy dioxane which is insoluble in the initial reaction solvent. The dioxane compound is separated from the initial reaction solvent and is subsequently catalytically hydrogenated to form trimethylene glycol. The patent generally discusses the possibility of using as the hydroformylation reaction catalyst, transition metals, particularly those of Group VIII of the Periodic Table, e.g., cobalt, iron, nickel, osmium, and complexes such as cobalt carbonyl tertiary phosphine and rhodium carbonyl. However, the examples in said patent are limited to the use of dicobalt octacarbonyl catalyst.

U.S. Patent No. 3,054,813 is directed toward a process for the production of 3-hydroxyaldehydes or alpha, beta-unsaturated aldehydes by the reaction of epoxides with synthesis gas. Said patent shows the use of a cobalt carbonyl catalyst for the hydroformylation of ethylene oxide, but the product which resulted was acrolein.

In an article by Yokokawa et al., Bulletin of the Chemical Society of Japan (Vol. 37, page 677, 1964), there is shown an attempt to hydroformylate ethylene oxide and propylene oxide using a cobalt carbonyl catalyst. In the case of ethylene oxide, the product was overwhelmingly composed of acetaldehyde. Small amounts of acrolein were formed. In the case of propylene oxide, under some conditions reasonable yield of 3-hydroxybutyraldehyde were produced, but the production of 1,3-butanediol is not mentioned.

It is likely that processes which produce 1,3-glycols from epoxides using "hydroformylation" catalysts, produce 3-hydroxyaldehydes as chemical intermediates which can either by hydrogenated to 1,3-glycols in situ, or isolated in some manner (as in the form of the aforementioned hydroxyalkyldioxanes) and then hydrogenated in a separate step. However, 3-hydroxyaldehydes, such as hydroacrylaldehyde, are unusually reactive species and readily undergo a variety of side reactions. In a literature review entitled "New Synthesis With Carbon Monoxide", B. Cornils, Springer Verlag, page 131, 1980, it was stated that numerous attempts had been made to subject oxiranes (epoxides) to the hydroformylation reaction to produce hydroxyaldehydes and that on account of the greater reactivity, not only of epoxides, but also of the resulting hydroxyaldehydes, the epoxide hydroformylation generally led to the formation of a mixture of products and thus unsatisfactory yields.

Under the conditions of a hydroformylation reaction, isomerization of ethylene oxide to acetaldehyde (which can be hydrogenated to ethanol) can occur. Furthermore, if hydroformylation of ethylene oxide to hydracrylaldehyde is successful, hydracryladehyde can dehydrate to yield acrolein, which can be hydrogenated to propanal or propanol, or the hydracryladehyde can undergo condensation (aldol) reactions with other aldehyde molecules to given C₆ branched aldehydes, which can undergo dehydration and hydrogenation reactions. It is therefore highly desirable that a catalyst for the production of 1,3-propanediol from ethylene oxide should be able to rapidly hydrogenate hydracryladehyde in situ before undesirable side reactions can occur. Such a catalyst needs also to have the economic advantage of producing the 1,3-propanediol product in a single reactor, without the need for a large and expensive apparatus for the isolation and subsequent hydrogenation of aldehydes.

Thus, there remains a need for an effective method for manufacturing 1,3-glycols, especially from epoxides, which process is usable in a commercial manner.

### SUMMARY OF THE INVENTION

It has now been discovered that epoxides may be converted into 1,3-glycols by a carbonylation reaction which uses rhodium as the catalyst. Thus, the present invention provides a process for manufacturing 1,3-glycols of the formula 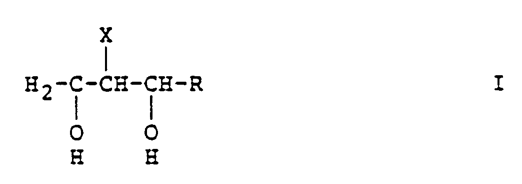
wherein R represents hydrogen, a monovalent aliphatic or aromatic group having from one to twelve carbon atoms, or a divalent aliphatic group having from 4 to 6 carbon atoms which together with X forms a cyclic structure, and X represents hydrogen, or if R is divalent, a bond with R. The process comprises reacting an epoxide of the formula 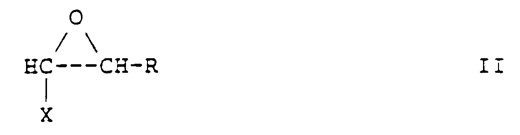
wherein R and X have the aformentioned meaning, with CO and H₂ in a suitable reaction solvent, wherein said process is characterized in that the reaction mixture contains (1) an epoxide of the foregoing structure at a concentration from 0.1 to 30 weight % (2) rhodium at a molar concentration from 0.00001 to 0.1 molar; (3) a phosphine having the formula

PR₁R₂R₃ III

wherein R₁, R₂, and R₃ are independently selected from the group consisting of aliphatic and aromatic hydrocarbon groups, the molar ratio of rhodium to phosphine being from 10:1 to 1:10; (4) water in an amount from 0.00 to 25 weight percent based on the weight of the reaction mixture; (5) CO; (6) H₂ and (7) an acid, the molar ratio of acid to phosphine being from 10:1 to 1:10; wherein the molar ratio of CO to H₂ is from 10:1 to 1:10, and wherein the reaction takes place at a temperature from 50 to 200°C under a pressure from 1480 000 to 69051 000 Pa (from 200 to 10,000 psig), for a period of time which is sufficient to form at least some of the desired 1,3-glycol.

### DETAILED DESCRIPTION OF THE PREFERRED EMBODIMENTS

As indicated above, the process of the present invention provides a method for the manufacture of 1,3-glycols through the carbonylation of epoxides. The desired glycols therefore contain one more carbon atom and one more oxygen atom than the epoxide reactant. Thus, for example, when the epoxide is ethylene oxide, containing 2 carbon atoms, the resultant 1,3-glycol is 1,3-propanediol, containing 3 carbon atoms. Examples of other specific epoxides which are useful in the present invention include propylene oxide, 1,2-epoxyoctane, cyclohexene oxide, and styrene oxide.

The epoxides, as indicated previously, have the general formula 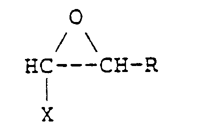
wherein R is hydrogen, a monovalent aliphatic or aromatic group having from one to twelve carbon atoms, or a divalent aliphatic group having from 4 to 6 carbon atoms which together with X forms a cyclic structure, and X represents hydrogen or, if R is divalent, a bond with R. R therefore may be a monolvalent alkyl group containing, for example, from one to six carbon atoms or may be a divalent alkyl group or an aromatic group, such as a phenyl group. If, for example, R is a divalent alkyl group having four carbon atoms, then the epoxide is cyclohexene oxide. The epoxide is usually present in the reaction mixture at a concentration of 0.1 to 30 weight percent. Typically the concentration of epoxide is from 1 to 20 weight percent.

The various epoxides may require different reaction conditions, to achieve optimum results in terms of product yield and selectivity, as well as different specific rhodium, phosphine, or acid components. Using the system rhodium, tricyclohexyl phosphine, and phosphoric acid, ethylene oxide gives good product yield and selectivity, and propylene oxide results in good product selectivity. Cyclohexene oxide and epoxyoctane give some products attributable to epoxide carbonylation. Conditions for the latter epoxides may possibly be optimized to achieve better product yield and selectivity.

The carbonylation reaction, as indicated previously, takes place in a suitable solvent. As a general principle, solvents which may be categorized as having medium to high polarity are suitable, such as aromatic solvents, ethers, polyethers, sulfones, and alcohols. Depending upon the reactivity of the particular solvent selected and the specific conditions to be employed, ketones, and esters may also be usable. The preferred solvents generally are high molecular weight ethers, polyethers, and cyclic ethers, especially glycol polyethers. An especially preferred solvent is tetraglyme, the dimethylether of tetraethylene glycol, 2,5,8,11,14-pentaoxapentadecane. Particularly useful solvents also include tetrahydrofuran, diglyme and Ucon™ oils, mixed glycol polyethers or ethylene and propylene glycol subunits.

To be suitable, a solvent should solubilize the epoxide reactant and should not adversely react with any of the components of the reaction mixture or the desired product. Thus, for lower molecular weight epoxides and glycols, solvents such as tetraglyme, tetrahydrofuran and the like are usually used. For higher molecular weight epoxides and glycols, hydrocarbon solvents such as petroleum ethers, toluene, and xylene may be appropriate. The latter solvents are less suitable for lower molecular weight epoxides and glycols such as ethylene oxide and 1,3-propanediol. Such hydrocarbon solvents are not suitable as the low molecular weight epoxides are insoluble therein and separate from such solvents.

The rhodium which is employed in the present process may be introduced in the form of rhodium metal, rhodium oxides, rhodium salts, and/or rhodium complexes. The only proviso is that the rhodium complex should not contain ligands which insolubilize or poison the catalyst. Thus, selection of the particular rhodium component may, in part, depend upon the solubility of the particular rhodium metal or compound in the specific solvent utilized as the reaction medium. The rhodium useful in the practice of the present invention includes rhodium metal, rhodium oxides, RhI₃, RhBr₃, RhCl₃, Rh(Acac)₃, Rh(CO)₂Acac, [RhCl(CO)₂]₂ and Rh(NO₃)₃, wherein Acac represents acetylacetonate. Likewise, the rhodium useful in the practice of the present invention may be a rhodium carbonyl-phosphine complex which has been preformed, prior to introduction into the reaction mixture, using any suitable technique for preforming such complexes. The concentration of the rhodium in the reaction solvent should be in the range from 0.00001 molar to 0.1 molar. Preferably, the concentration of rhodium will be from 0.005 to 0.05 molar.

The phosphine which is employed in the present invention has the general formula

PR₁R₂R₃

wherein R₁, R₂, and R₃ are all independently selected from the group consisting of aliphatic and aromatic radicals. Preferably, R₁, R₂, and R₃ are all alkyl groups containing from 1 to 12 carbon atoms. Particularly preferred alkyl groups include n-propyl, i-propyl, n-butyl, i-butyl, and cyclohexyl. Aryl and mixed aryl/alkyl phosphines may be used in the present invention, but their efficacy is dependent upon the particular reaction conditions, including solvent, which is employed. In general, the aryl and mixed aryl/alkyl phosphines are not as efficacious as the trialkyl phosphines. The most preferred phosphine is tricyclohexyl phosphine. Triisopropyl phosphine and triisobutyl phosphine have also been found to be extremely useful.

The amount of phosphine employed is not critical, but in general, it has been found that a molar ratio of rhodium to phosphine of 1:1 is preferred. Broadly, a range of 10:1 to 1:10 is operable, however. Typically, the molar ratio of rhodium to phosphine will be from 2:1 to 1:2.

The presence of acid in the reaction mixture has been found to be useful in achieving optimum results. Usually medium and strong acids are preferable for use in the present process. Some acids are, however, less desirable because of their corrosiveness or due to their insolubility, especially of the corresponding anion, in the particular solvent being employed. Halides in particular may promote decomposition of ether solvents and would thus be less desirable in such solvents. However, HI and HCl are useful acids in the process. Preferable acids include phosphoric acid and para-toluene sulfonic acid. Weaker acids such as acetic may be operable, depending upon the particular reaction conditions, but may also become esterified.

Usually the amount of acid, if present, will be approximately in an equal molar amount with the rhodium and/or phosphine. When an acid is employed, the preferable molar ratio of rhodium:phosphine:acid, is approximately 1:1:1. As a general principle, it has been found that variation of the molar ratio by factors of 2-5 only result in mildly deleterious effects. Typically the molar ratio of acid to phosphine should not be greater than 5:1. It is believed that the presence of an acid is important to minimize by-product formation. Thus, the acid when present in sufficient quantity will tend to neutralize strongly basic components of the medium which is important because in a basic environment aldehydes tend to undergo condensation reactions and thus to form undesirable by-products.

The ratio of hydrogen to carbon monoxide employed in the hydroformylation reaction has not been found to be critical. In general, a ratio of H₂:CO from 10:1 to 1:10 is operable. Typically, a ratio from 5:1 to 1:1 is employed. As a general matter, it has been found that yields increase with increasing H₂:CO ratios.

With respect to the pressure employed during the hydroformylation reaction, the pressure is not critical and generally falls within the range from 1 480 000 to 69 051 000 Pa (from 200 to 10,000 psig). Preferably, the pressure falls in the range of from 6 996 000 to 20 786 000 Pa (from 1,000 to 4,000 psig). It has been found, generally, that both rates and selectivities are promoted by increased hydrogen partial pressure. However, increasing carbon monoxide partial pressure may be detrimental.

With respect to the conversion of ethylene oxide to 1,3-propanediol, the rate of product formation increases by a factor of 2-3, as hydrogen partial pressure is increased from 3 549 000 to 13 891 000 Pa (from 500 to 2,000 psig). As carbon monoxide partial pressure is increased from 3 549 000 to 6 996 000 Pa (from 500 to 1,000 psig), there appears to be a small rise in the rate of product formation, but as carbon monoxide partial pressure is increased further, from 6 996 000 to 13 891 000 Pa (from 1,000 to 2,000 psig), the rate of 1,3-propanediol formation drops sharply.

With respect to the effect of pressure on the efficiency of the conversion to 1,3-propanediol, the trends are similar to those related to conversion rates. Thus, as hydrogen partial pressure is increased from 3 549 000 to 6 996 000 Pa (from 500 to 1,000 psig), and then to 13 891 000 Pa (2,000 psig), the 1,3-propanediol selectivity traumatically increases, while selectivity to C₂ and C₃ products decreases.

The temperature used in the carbonylation reaction also is not critical. As a general proposition, it has been found that increasing temperature also increases rates. However, increasing temperatures have an adverse affect on selectivity. Thus, some balancing of temperature is required in order to achieve suitable rates and suitable selectivities. Generally, a temperature of from 50 to 200°C will be employed, preferably from 100 to 150°C.

For the production of 1,3-propanediol, using the rhodium, tricyclohexylphosphine, phosphoric acid system, at 2500 psig in tetraglyme solvent, containing 0.69 M water, a temperature of 110°C results in an efficiency of 85 to 90%, but by increasing the temperature to 130°C, the efficiency is reduced to 70 to 75%. Also, as the temperature increases, the induction period also declines.

As a general proposition with respect to H₂:CO composition, reaction pressure, and reaction temperature, all will vary somewhat based upon the particular reaction conditions employed and adjustment thereof is within the ordinary skill of one in the art.

Water, in general, has been found to be useful in conjunction with many catalysts and solvent/acid combinations. In particular, though the presence of water is not necessary for the function of the catalysts used in this invention, in the absence of water, substantial induction periods are sometimes observed between the injection of ethylene oxide and the onset of the uptake of synthesis gas and the production of product, such as 1,3-propanediol. It has been found that the presence of small amounts of water can sometimes substantially decrease the length of the induction periods, and hence shorten the overall reaction times. However, if the amount of water is increased beyond a given level, poorer yields may result. As a broad proposition, from 0.00 to 25 wt.% water is employed, preferably from 0.0 to 10 wt.%. The amount of water employed, as indicated, to achieve optimum results, will vary depending upon the particular reaction system and conditions employed.

With respect of the production of 1,3-propanediol, using the system comprised of rhodium, tricyclohexlyphosphine, and phosphoric acid catalyst, at 110°C, 2500 psig pressure and a 2:1 ratio of hydrogen to carbon monoxide in tetraglymate solvent, it has been found that by reducing the water concentration from 3.47 M down to 0, the conversion efficiency improved substantially, from 70-75% to 85-88%. Although some efficiency improvement would be expected due to the reduction in losses caused by ethylene oxide hydrolysis, the reasons for such a large efficiency increase are unknown and quite unexpected. Likewise, the reduction in the induction period, the time required before synthesis gas uptake occurs, after ethylene oxide injection, is also unexpectedly increased. Thus, at a water concentration of 3.47 M, the induction period is typically on the order of 30 to 40 minutes, with a total run time of 4 to 5 hours. The induction time is increased only slightly as the concentration of water is altered down to 0.69 M. However, as further reductions in the concentration of water are made, the induction periods dramatically lengthen up to approximately 3 hours. Of importance is the fact that after synthesis gas uptake begins, regardless of the concentration of water initially present, the rates of synthesis gas uptake are roughly equivalent.

The present invention is capable of achieving yields of 1,3-glycols, such as 1,3-propanediol in excess of 85 percent based upon the epoxide, such as ethylene oxide, and production rates as high as 0.92 mols/liter/hr. in a single carbonylation reactor. Such results are certainly unexpected and surprising, since the use of rhodium catalysts for the carbonylation of epoxides to 1,3-glycols has not been shown in the prior art. The present results are also surprising in view of the fact that most prior art cobalt catalysts generally only achieved substantially lower rates and efficiencies for precursors of 1,3-propanediol, while the present process provides a high yield, single step process for the production of 1,3-glycols without the need for separate, large hydrogenation reactors for 1,3-glycol precursors.

The present invention is further shown by the following non-limiting examples.

### General Experimental Method Employed In The Examples

All examples were performed in a batch autoclave unit which consisted of a 300 cc Hastelloy autoclave, equipped with remotely operable controls for feeds, vents, stirring, heating, cooling, and the like. Standard stainless-steel tubing and Swagelok fittings were employed at the lower reactor pressures. At pressures of 2500 psig, high-pressure type fittings, valves, and tubings were employed.

All catalysts and solvents were weighed under nitrogen and rapidly charged to a cold autoclave which was then purged twice with nitrogen and twice with synthesis gas. Subsequently, the autoclave was pressurized with synthesis gas to the desired pressure and heated under slow stirring to reaction temperature, over a period of 0.5 to 4.0 hours. Ethylene oxide was then injected into the autoclave from either a pressurized blowcase bomb or a Ruska syringe pump, at which time fast stirring was commenced and the total reactor pressure raised to the final desired value, using synthesis gas to control the pressure. Constant reactor pressures were maintained automatically during the runs by feeding synthesis gas on demand from a high-pressure synthesis gas reservoir of known volume. The uptake of reaction synthesis gas was monitored by periodic measurement of the pressure of the synthesis gas reservoir. Runs were terminated, usually when synthesis gas uptake slowed to nearly zero, by slowing the stirring rate, terminating the synthesis gas feed, and cooling the reactor as rapidly as possible, typically over a 30 to 60 minute period.

Small quantities of ethylene oxide were injected into the reactor which was hot and pressurized, using either a Ruska syringe or a pressurized blowcase bomb. When the latter method was employed, the ethylene oxide was charged to the blowcase bomb by condensation of ethylene oxide vapor, from a lecture bottle, into the blowcase which was chilled to dry ice temperatures. When ethylene oxide had been charged to the blowcase, the blowcase was detached from the transfer apparatus, weighed, then connected to the autoclave. Synthesis gas pressure was used to force the ethylene oxide from the blowcase into the autoclave.

When the Ruska pump method was used for injecting the ethylene oxide, liquid ethylene oxide was transferred through stainless steel lines to the Ruska syringe pump which then injected the ethylene oxide into the autoclave unit.

Because liquid ethylene oxide became held up in the lines, fittings, and valves leading to the autoclave, it was necessary to charge somewhat larger than theoretical quantities of ethylene oxide to the blowcase or Ruska pump and then calibrate the unit for the quantity of ethylene oxide which actually reached the autoclave. Calibration runs were performed by charging the reactor with 100 grams of water and 1.8 grams of sulfuric acid and heating it to 100°C. Ethylene oxide was then charged to the blowcase or Ruska pump, injected into the reactor, which was then heated for two hours to achieve ethylene oxide hydrolysis to ethylene glycol. The resulting ethylene glycol:water solutions were analyzed for ethylene glycol using gas chromatography. In a typical run, 12.0 grams of ethylene oxide would be charged to the blowcase and the ethylene glycol equivalent of 10.0 grams of ethylene oxide reached the reactor. Ethylene oxide feed was then back-calculated from the ethylene glycol and plots of ethylene oxide observed versus ethylene oxide charged, were constructed. Such plots were found to be reasonably linear over the range of 5 to 15 grams of ethylene oxide and typically showed 75 to 85 percent ethylene oxide efficiency in the transfer operation. The results of such calibration runs were then used to calculate ethylene oxide feed for the catalytic carbonylation runs.

With respect to the materials employed in the Examples, [RhCl(CO)₂]₂, P(C₆H₁₁)₃ and p(n-C₄H₉)₃ were purchased from Strem Chemicals and stored and handled under nitrogen. Rh(CO)₂Acac was either purchased fron Englehard or prepared from RhCl₃ 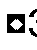 3H₂O, acetyl acetone, and dimethylformamide and then recrystallized from hexane to yield green-red crystalline needles.

Ethylene oxide (99.7% min) was purchased from Matheson and stored in chilled water. H₂/CO mixtures were purchased from Iweco. Tetraglyme and sulfolane were used in the Examples as received from Aldrich, as was the n-butanol which was received from Burdick and Jackson. The toluene and tetrahydrofuran which was received from Aldrich, were distilled from sodium medal under nitrogen.

In the accompanying examples, the reaction products are sometimes divided into four categories. "PDO Precursors" consist of the sum of 1,3-propanediol (1,3-PDO), 3-hydroxypropionaldehyde (HPA), and 2-hydroxyethyle-1,3-dioxane (HED). The products "PDO Precursors" include, in addition to 1,3-PDO, HPA and HED because, presumably, they could be hydrolyzed and/or hydrogenated to 1,3-PDO. "EtOH Precursors" include the sum of acetaldehyde and ethanol. "PrOH Precursors" include the sum of acrolein, propionaldehyde, and propanol. "Others" consists of all the identified products not falling within one of the foregoing categories. In examples where yields are quoted, yields were calculated from the observed moles of product divided by the moles of EO calculated (via use of the EO calibration procedure) to have been charged to the reactor. In some cases, due to some experimental error in the ethylene oxide injection procedure and the associated calibration procedure, the sum of analyzed products derived from ethylene oxide somewhat exceeded the calculated quantity of ethylene oxide charged to the reactor. In those examples, product efficiencies (which have been normalized to 100% EO accountability) are quoted.

### EXAMPLE 1

Eighty grams of tetraglyme, 0.50 gram of Rh(CO)₂Acac, 1.1 grams of tricyclohexylphosphine, and 0.1 gram of hydroquinone were charged to a 300 cc autoclave according to standard procedures. The mixture was heated to 100°C under 6 996 325 Pa (1000 psig) of 2:1 H2/CO, the reactor pressure then increased to 1400 psig and 8.9 grams of ethylene oxide was injected from a Ruska pump. Uptake of 2:1 H₂/CO gas began in less than 25 minutes, and the pressure was thereafter maintained at approximately 9 754 000 Pa (1400 psig) by addition of 2:1 H₂/CO on demand. The reaction was terminated after 4.6 hours (gas adsorption had not completely ceased), and the product removed and analyzed by gas chromatography. The product contained no free 1,3-propanediol, but did contain small amounts of HPA (0.0016 mole) and HED (0.0002 mole). The major products were unconverted ethylene oxide (0.0895 mole), acrolein (0.0174 mole), ethylene glycol (0.0112 mole), and 2-methyl-pentanol (0.0097 mole). Smaller amounts of acetaldehyde, propanol, and 2-methyl pentanal were also detected, along with numerous other small byproducts. This example illustrates that ethylene oxide is carbonylated to C₃ products by the use of Rh/phosphine catalysts in the absence of acid promoters. Only small amounts "PDO-precursor" molecules were produced, but substantial amounts of C₃ products (which presumably arise from the reaction of EO with CO and H₂) are observed. Also, substantial amounts of C₆ products (which may arise from the coupling of C₃ aldehydes) were produced.

### EXAMPLE 2

Eighty grams of tetraglyme, 0.53 gram of Rh(CO)₂Acac, 0.54 grams of tricyclohexylphosphine, 1.0 gram of 57% aqueous HI, 5.0 grams of H₂O, and 0.1 gram of hydroquinone were charged to an autoclave and heated to 120°C under 10 444 000 Pa (1500 psig) of 2:1 H₂/CO. Ethylene oxide (8.9 grams) was injected from a Ruska pump and thereafter 2:1 H₂/CO was fed on demand to maintain a 1500 psig pressure. The run was terminated after 116 minutes. The product contained 0.057 mole of 1,3-PDO, 0.014 mole of HPA, and 0.012 mole of HED, for a total yield of "PDO Precursors" of 47.4%. Major byproducts included Ethanol (0.045 mole), acetaldehyde (0.019 mole), propanal (0.009 moles), propanol (0.004 mole), 2-methyl-pentanal (0.006 mole) and 2-methylpentanol (0.005 mole). Traces of ethylene glycol and other unidentified products were also observed. This example illustrates the use of an acid promoter (HI) to improve the activity and selectivity of a rhodium/phosphine catalyst for the carbonylation of ethylene oxide to 1,3-propanediol at relatively low pressures.

### EXAMPLE 3

Eighty grams of tetraglyme, 0.54 gram of rhodium trichloride hydrate, 0.53 gram of tricyclohexylphosphine, 0.20 gram of concentrated aqueous HCl, 5.11 grams of H₂O, and 0.11 gram of hydroquinone were charged to an autoclave. The mixture was heated to 120°C under 6 996 000 Pa (1000 psig) of 2:1 H₂/CO, and 9.4 grams of ethylene oxide was injected from a blowcase bomb utilizing 2:1 H₂/CO, and the final pressure brought to 17 339 000 Pa (2500 psig). Gas uptake began after approximately 50 minutes, and the run was terminated after 3 hours when gas uptake had essentially stopped. The product was found to contain 0.1506 mole of 1,3-PDO and 0.006 mole of HPA, for a combined yield of 73.4%. Major byproducts included ethanol (0.053 mole), propanol (0.017 mole), ethylene glycol (0.013 mole) and ethylene oxide (0.003 mole). Because the apparent yield of EO derived molecules was 114.5%, the ethylene oxide efficiencies were normalized, and showed that "PDO Precursors" were formed with a 64.9% efficiency at a rate of 0.626 moles/liter/hour. This example illustrates that 1,3-propanediol can be produced in reasonably good efficiency from a catalyst precursor which does not contain acetylacetonate ligands. It also illustrates the use of hydrochloric acid promoter.

### EXAMPLE 4

Eighty grams of tetraglyme, 0.51 gram of Rh(CO)₂Acac, 0.41 gram of triisobutylphosphine, 0.14 gram of phosphoric acid, 5.11 gram of H₂O, and 0.1 gram of hydroquinone were charged to an autoclave. The mixture was heated to 120°C under 6 996 000 Pa (1000 psig) of 1:1 H₂/CO, then 10.0 grams of ethylene oxide was injected from a blowcase bomb, and the pressure increased to 17 339 000 Pa (2500 psig). Uptake of synthesis gas began after about 40 minutes, and the run was terminated after 3.6 hours when syngas uptake had essentially stopped. Analysis of the product showed the presence of 0.166 mole of 1,3-PDO. Major byproducts included ethanol (0.0184 mole), acetaldehyde (0.0053 mole), ethylene glycol (0.012 mole), propanal (0.006 mole) and propanol (0.011 mole). The yield of 1,3-propanediol was 74.3% at a rate of 0.567 mole/liter/hour. This example illustrates that 1,3-propanediol can be produced in good efficiency by use of a trialkylphosphine other than tricyclohexylphosphine.

### EXAMPLE 5

Eighty grams of tetraglyme, 0.52 gram of Rh(CO)₂Acac, 0.54 grams of dicyclohexylphenylphosphine, 0.14 gram of phosphoric acid, 5.1 grams of H₂O, and 0.1 grams of hydroquinone were charged to the autoclave and heated to 110°C under 6 996 000 Pa (1000 psig) of 2:1 H₂/CO. Ethylene oxide (10.1 grams) was injected from a blowcase bomb, and the pressure increase 17 339 000 Pa (2500 psig). Gas uptake began after about 1 hour, and the run was terminated after 6.5 hours. Analysis of the product showed the presence of 0.115 mole of 1,3-propanediol, 0.040 mole of acetaldehyde, 0.027 mole of ethanol, and smaller amounts of acrolein, propanal, propanol, and ethylene glycol. The yield of 1,3-propanediol was calculated to be 50%, at a rate of 0.22 mole/liter/hour. This example illustrates that 1,3-propanediol can be produced in reasonable yields by use of a rhodium catalyst promoted by a phosphine which contains an aryl substituent.

### EXAMPLE 6

Eighty grams of tetraglyme, 0.51 gram of Rh(CO)₂Acac, 0.53 gram of tricyclohexylphosphine, 0.13 gram of phosphoric acid, 5.0 grams of H₂O, and 0.1 grams of hydroquinone were charged to an autoclave and heated to 110°C under 6 996 000 Pa (1000 psig) of 2:1 H₂/CO. Ethylene oxide (10.2 grams) was injected from a blowcase bomb, and the pressure increased to 17 339 000 Pa (2500 psig). Gas uptake began after 50 minutes, and the run was terminated after 4.0 hours. The product contained 0.1905 mole of 1,3-propanediol, 0.0235 mole of ethanol, 0.0154 mole of propanol, 0.0125 mole of ethylene glycol, and 0.0089 mole of ethylene oxide. Because the apparent yield of EO derived molecules was 110%, the ethylene oxide based efficiencies were normalized, and showed that 1,3-propanediol was formed with a 77.5% efficiency at a rate of 0.595 mole/liter/hour. This example illustrates that 1,3-propanediol can be produced in relatively good efficiencies and rates by the use of a rhodium/phosphine catalyst promoted by water and phosphoric acid.

### EXAMPLE 7

Eighty grams of tetraglyme, 0.52 gram of Rh(CO)₂Acac, 0.53 gram of tricyclohexylphosphine, 0.13 gram of phosphoric acid, 1.05 grams of H₂O, and 0.10 grams of hydroquinone were charged to an autoclave and heated to 110°C under 6 996 000 Pa (1000 psig) of 2:1 H₂/CO. Ethylene oxide (10.0 grams) was injected from a blowcase bomb, and the pressure increased to 17 339 000 Pa (2500 psig). Gas uptake began after approximately 90 minutes, and the run was terminated after 5.5 hours. The product contained 0.1931 mole of 1,3-propanediol, 0.0172 mole of ethanol, 0.0141 mole of propanol, and 0.0029 mole of ethylene oxide. Overall, 1,3-propanediol was produced in 85% yield at a rate of 0.438 mole/liter/hour. This example illustrates that 1,3-propanediol efficiencies are somewhat improved when the amount of water is decreased relative to the levels used in previous examples.

### EXAMPLE 8

Eighty grams of tetraglyme, 0.52 gram of Rh(CO)₂Acac, 0.54 gram of tricyclohexylphosphine, 0.14 gram of phosphoric acid, and 0.10 gram of hydroquinone were charged to the autoclave. No water was added to the charge. The mixture was heated to 110°C under 6 996 000 Pa (1000 psig) of 2:1 H₂/CO. Ethylene oxide (10.0 grams) was injected from a blowcase bomb, and the pressure increased to 17 339 000 Pa (2500 psig). Gas uptake began after approximately 160 minutes, and the run was terminated after 6.5 hours. Product analysis shoved the presence of 0.1983 mole of 1,3-propanediol, 0.0166 mole of ethanol, 0.0046 mole of acetaldehyde, and 0.0092 mole of propanol. The yield of 1,3-propanediol was 87% at a rate of 0.377 mole/liter/hour. This example illustrates that 1,3-propanediol can be produced in good yields in the absence of water promoter, though "induction periods" are somewhat longer in the absence of water.

### EXAMPLE 9

Eighty grams of tetraglyme, 0.51 gram of Rh(CO)₂Acac, 0.54 grams of tricyclohexylphosphine, 0.13 gram of phosphoric acid, and 1.05 grams of H₂0 were charged to the autoclave. No hydroquinone was added. The mixture was heated to 110°C under 6 996 000 Pa (1000 psig) of 2:1 H₂/CO and ethylene oxide (10.1 grams) was injected from a blowcase bomb and the pressure increased 17 339 000 Pa (2500 psig). Gas uptake began after about 90 minutes, and the run was terminated after 6.5 hours. Product analysis showed the presence of 0.2172 mole of 1,3-propanediol, 0.0196 mole of ethanol, 0.0133 mole of propanol, and traces of acetaldehyde and ethylene oxide. Because the apparent yield of ethylene oxide derived molecules was 113%, the ethylene oxide efficiencies were normalized, and showed that 1,3-propanediol was produced in 85% efficiency at a rate of 0.41 mole/liter/hour. This example illustrates that the absence of hydroquinone "promoter" (which was included in many of the examples documented here) has no substantial effect on the yields or rates of production of 1,3-propanediol.

### EXAMPLE 10

Eighty grams of Ucon 50-HB-100 polyglycol ether, 0.52 gram of Rh(CO)₂Acac, 0.53 gram of tricyclohexylphosphine, 0.13 gram of phosphoric acid, 5.1 grams of H₂O, and 0.1 grams of hydroquinone were charged to the autoclave and heated to 110°C under 6 996 000 Pa (1000 psig) of 2:1 H₂/CO. Ethylene oxide (10.0 grams) was injected from a blowcase bomb, and the pressure increased to 17 339 000 Pa (2500 psig). Gas uptake began after approximately 20 minutes, and the run was terminated after 3.0 hours. Product analysis showed the presence of 0.1775 mole of 1,3-propanediol, 0.0243 mole of propanol, 0.0269 mole of ethanol, 0.0125 mole of acetaldehyde, 0.0102 mole of acrolein, and traces of ethylene oxide and ethylene glycol. Because the apparent yield of ethylene oxide derived molecules was 112%, the ethylene oxide efficiencies were normalized, and showed that 1,3-propanediol was produced in 70.2% efficiency, at a rate of 0.717 mole/liter/hour. This example illustrates that 1,3-propanediol can be produced in good efficiencies in a complex polyglycol ether solvent other than tetraglyme.

### EXAMPLE 11

Eighty grams of tetrahydrofuran, 0.52 gram of Rh(CO)₂Acac, 0.54 gram of tricyclohexylphosphine, 0.13 gram of phosphoric acid, 5.1 grams of H₂O, and 0.10 gram of hydroquinone were charged to the autoclave and heated 110°C under 6 996 000 Pa (1000 psig) of 2:1 H₂/CO. Ethylene oxide (10.0 grams) was injected from a blowcase bomb, and the pressure increased to 17 339 000 Pa (2500 psig). Gas uptake began after approximately 40 minutes, and the run was terminated after 3.5 hours. Analysis of the product showed the presence of 0.2058 mole of 1,3-propanediol, 0.0166 mole of ethanol, 0.0114 mole of propanol, and 0.0026 mole of acrolein. Because the apparent yield of ethylene oxide derived molecules was 106%, the ethylene oxide efficiencies were normalized to show that 1,3-propanediol was produced in 85.8% efficiency at a rate of 0.739 mole/liter/hour. This example illustrates that 1,3-propanediol can be produced in high efficiency in a monoether solvent, and that is extremely unlikely that 1,3-propanediol could be derived from decomposition of solvent.

### EXAMPLE 12

Eighty grams of tetraglyme, 0.51 gram of Rh(CO)₂Acac, 0.54 gram of tricyclohexylphosphine, 0.14 gram of phosphoric acid, 5.11 grams of H₂O, and 0.11 gram of hydroquinone were charged to the autoclave and heated to 110°C under 6 996 000 Pa (1000 psig) of 2:1 H₂/CO. Propylene oxide (15.8 grams) was charged to a blowcase, and then injected into the autoclave. No attempt was made to callibrate for losses of propylene oxide during the injection procedure. Gas uptake began after 2.5 hours, and the run was terminated after 7.5 hours before gas uptake had stopped. The liquid product was recovered (101.7 grams) and analyzed by GC and GC-mass spectra. The product was found to contain approximately 7.8 wt% of unconverted propylene oxide, approximately 5.5 wt% 1,3-butanediol, and approximately 0.5 wt% of 1,2-propylene glycol. No other significant products were detected. Although not optimized, this example illustrates that 1,3-propanediol can be produced via carbonylation of propylene oxide in a relatively selective manner.

## Claims

1. A process for manufacturing 1,3-glycols of the formula 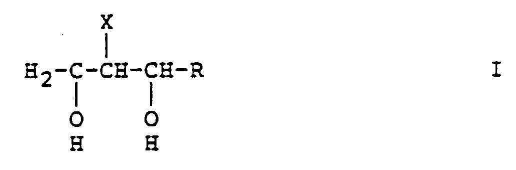 wherein R represents hydrogen, a monovalent aliphatic or aromatic group having from one to twelve carbon atoms, or a divalent aliphatic group having from 4 to 6 carbon atoms which together with X forms a cyclic structure, and X represents hydrogen, or if R is divalent, a bond with R, wherein said process comprises reacting an epoxide of the formula 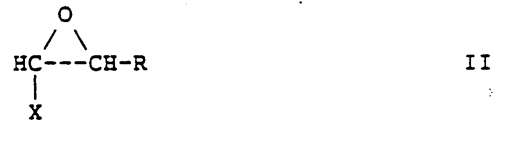 wherein R and X have the aformentioned meaning, with CO and H₂ in a suitable reaction solvent, said process being characterized in that the reaction mixture comprises (1) an epoxide of the foregoing structure at a concentration from 0.1 to 30 weight percent; (2) rhodium at a molar concentration from 0.00001 to 0.1 molar; (3) a phosphine having the formula
PR₁R₂R₃ III
wherein R₁, R₂, and R₃ are independently selected from the group consisting of aliphatic and aromatic hydrocarbon groups, the molar ratio of rhodium to phosphine being from 10:1 to 1:10; (4) water in an amount from 0.00 to 25 weight percent based on the weight of the reaction mixture; (5) CO; (6) H₂; and (7) an acid, the molar ratio of acid to phosphine being from 10:1 to 1:10; wherein the molar ratio of CO to H₂ is from 10:1 to 1:10, and wherein the reaction takes place at a temperature from 50 to 200°C under a pressure from 1 480 000 to 69 051 000 Pa (from 200 to 10,000 psig), for a period of time which is sufficient to form at least some of the desired 1,3-glycol.

2. The process of claim 1 wherein the acid is selected from the group consisting of HI, HCl, paratoluene sulfonic acid and phosphoric acid.

3. The process claim 2 wherein the epoxide is selected from the group consisting of ethylene oxide, propylene oxide, octene oxide, and cyclohexene oxide.

4. The process of claim 3 wherein the solvent is an ether or a mixture thereof.

5. The process of claim 4 wherein the solvent is selected from the group consisting of tetraglyme, tetrahydrofuran, and a mixture of glycol polyethers of ethylene and propylene glycols.

6. The process of claim 2 wherein the rhodium is selected from the group consisting of rhodium metal, rhodium oxides, RhI₃, RhBr₃, RhCl₃, Rh(Acac)₃, Rh(CO)₂Acac, [RhCl(CO)₂]₂, and Rh(NO₃)₃.

7. The process of claim 6 wherein the rhodium is present at a concentration from 0.005 to 0.10 molar.

8. The process of claim 7 wherein the phosphine is a trialkyl phosphine.

9. The process of claim 8 wherein the phosphine is selected from the group consisting of tricyclohexyl phosphine, triisopropyl phosphine, triisobutyl phosphine, tri-n-butyl phosphine, and tri-n-propyl phosphine.

10. The process of claim 9 wherein the molar ratio of rhodium to phosphine is from 1:2 to 2:1.

11. The process of claim 10 wherein the molar ratio of acid to phosphine is from 5:1 to 1:5.

12. The process of claim 11 wherein the ratio of H₂:CO is from 5:1 to 1:1.

13. The process of claim 1 wherein the 1,3-glycol is 1,3-propanediol, the epoxide is ethylene oxide, the solvent is tetraglyme, the reaction temperature is from 100 to 130°C, and the reaction pressure is from 6 996 000 to 20 786 000 Pa (from 1000 to 3000 psig).

## Patentansprüche

1. Verfahren zur Herstellung von 1,3-Glycolen der Formel 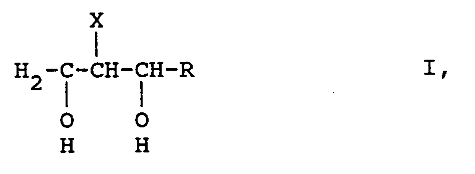 in der
R Wasserstoff, eine einwertige aliphatische oder aromatische Gruppe mit 1 bis 12 Kohlenstoff-Atomen oder eine zweiwertige aliphatische Gruppe mit 4 bis 6 Kohlenstoff-Atomen darstellt, die zusammen mit X eine cyclische Struktur bildet, und
X Wasserstoff oder, wenn R zweiwertig ist, eine Bindung mit R darstellt,
umfassend die Umsetzung eines Epoxids der Formel 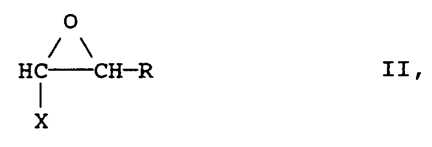 in der R und X die vorstehenden Bedeutungen haben, mit CO und H₂ in einem geeigneten Reaktionslösungsmittel, dadurch gekennzeichnet, daß die Reaktionsmischung
(1) ein Epoxid der vorstehenden Struktur in einer Konzentration von 0,1 bis 30 Gew.-%;
(2) Rhodium in einer Stoffmengen-Konzentration von 0,00001 bis 0,1-molar;
(3) ein Phosphin der Formel
PR₁R₂R₃ III,
in der R₁, R₂ und R₃ unabhängig aus der aus aliphatischen und aromatischen Kohlenwasserstoff-Gruppen bestehenden Gruppe ausgewählt sind, wobei das Stoffmengen-Verhältnis Rhodium zu Phosphin 10 : 1 bis 1 : 10 beträgt;
(4) Wasser in einer Menge von 0,00 bis 25 Gew.-%, bezogen auf das Gewicht der Reaktionsmischung;
(5) CO;
(6) H₂; und
(7) eine Säure
umfaßt, wobei das Stoffmengen-Verhältnis der Säure zu dem Phosphin 10 : 1 bis 1 : 10 beträgt, wobei das Stoffmengen-Verhältnis CO zu H₂ 10 : 1 bis 1 : 10 beträgt und wobei die Reaktion bei einer Temperatur von 50 °C bis 200 °C unter einem Druck von 1 480 000 bis 69 051 000 Pa (200 bis 10 000 psi) während einer Zeitspanne stattfindet, die ausreicht, um wenigstens eine gewisse Menge des gewünschten 1,3-Glycols zu bilden.

2. Verfahren nach Anspruch 1, worin die Säure aus der aus HI, HCl, p-Toluolsulfonsäure und Phosphorsäure bestehenden Gruppe ausgewählt ist.

3. Verfahren nach Anspruch 2, worin das Epoxid aus der aus Ethylenoxid, Propylenoxid, Octenoxid und Cyclohexenoxid bestehenden Gruppe ausgewählt ist.

4. Verfahren nach Anspruch 3, worin das Lösungsmittel ein Ether oder ein Gemisch aus solchen ist.

5. Verfahren nach Anspruch 4, worin das Lösungsmittel aus der aus Tetraglyme, Tetrahydrofuran und einem Gemisch aus Glycolpolyethern von Ethylen- und Propylenglycolen ausgewählt ist.

6. Verfahren nach Anspruch 2, worin das Rhodium aus der aus Rhodium-Metall, Rhodiumoxiden, RhI₃, RhBr₃, RhCl₃, Rh(Acac)₃, Rh(CO)₂Acac, [RhCl(CO)₂]₂ und Rh(NO₃)₃ bestehenden Gruppe ausgewählt ist.

7. Verfahren nach Anspruch 6, worin das Rhodium in einer Konzentration von 0,005 bis 0,10 molar vorliegt.

8. Verfahren nach Anspruch 7, worin das Phosphin ein Trialkylphosphin ist.

9. Verfahren nach Anspruch 8, worin das Phosphin aus der aus Tricyclohexylphosphin, Triisopropylphosphin, Triisobutylphosphin, Tri-n-butylphosphin und Tri-n-propylphosphin bestehenden Gruppe ausgewählt ist.

10. Verfahren nach Anspruch 9, worin das Stoffmengen-Verhältnis Rhodium zu Phosphin 1 : 2 bis 2 : 1 beträgt.

11. Verfahren nach Anspruch 10, worin das Stoffmengen-Verhältnis der Säure zu dem Phosphin 5 : 1 bis 1 : 5 beträgt.

12. Verfahren nach Anspruch 11, worin das Stoffmengen-Verhältnis H₂ : CO 5 : 1 bis 1 : 1 beträgt.

13. Verfahren nach Anspruch 1, worin das 1,3-Glycol 1,3-Propandiol ist, das Epoxid Ethylenoxid ist, das Lösungsmittel Tetraglyme ist, die Reaktionstemperatur 100 °C bis 130 °C beträgt und der Reaktionsdruck 6 996 000 bis 20 786 000 Pa (1000 bis 3000 psig) beträgt.

## Revendications

1. Procédé de production de 1,3-glycols de formule 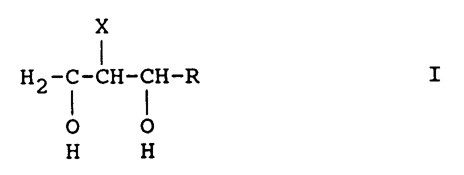 dans laquelle R représente l'hydrogène, un groupe aliphatique ou aromatique monovalent ayant 1 à 12 atomes de carbone ou un groupe aliphatique divalent ayant 4 à 6 atomes de carbone, qui forme conjointement avec X une structure cyclique, et X représente l'hydrogène, ou bien si R est divalent, une liaison avec R, ledit procédé comprenant la réaction d'un époxyde de formule 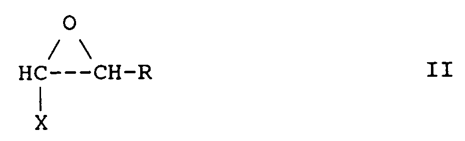 dans laquelle R et X ont les définitions indiquées ci-dessus, avec CO et H₂ dans un solvant réactionnel convenable, ledit procédé étant caractérisé en ce que le mélange réactionnel comprend (1) un époxyde de la structure ci-dessus à une concentration de 0,1 à 30 % en poids ; (2) du rhodium à une concentration molaire de 0,00001 à 0,1 M ; (3) une phosphine répondant à la formule
PR₁R₂R₃ III
dans laquelle R₁, R₂ et R₃ sont choisis indépendamment entre des groupes hydrocarbonés aliphatiques et aromatiques, le rapport molaire du rhodium à la phosphine étant compris entre 10:1 et 1:10 ; (4) de l'eau en une quantité de 0,00 à 25 % en poids sur la base du poids du mélange réactionnel ; (5) du CO ; (6) du H₂ ; et (7) un acide, le rapport molaire de l'acide à la phosphine étant compris entre 10:1 et 1:10 ; le rapport molaire de CO à H₂ ayant une valeur de 10:1 à 1:10 et la réaction ayant lieu à une température de 50 à 200°C sous une pression manométrique de 1 480 000 à 69 051 000 Pa (200 à 10 000 lb/in²) pendant une période qui est suffisante pour former au moins une certaine quantité du 1,3-glycol désiré.

2. Procédé suivant la revendication 1, dans lequel l'acide est choisi dans le groupe comprenant HI, HCl, l'acide paratoluène-sulfonique et l'acide phosphorique.

3. Procédé suivant la revendication 2, dans lequel l'époxyde est choisi dans le groupe comprenant l'oxyde d'éthylène, l'oxyde de propylène, l'oxyde d'octène et l'oxyde de cyclohexène.

4. Procédé suivant la revendication 3, dans lequel le solvant est un éther ou un mélange d'éthers.

5. Procédé suivant la revendication 4, dans lequel le solvant est choisi dans le groupe comprenant le tétraglyme, le tétrahydrofuranne et un mélange de polyéthers de glycol et d'éthylène- et de propylène-glycols.

6. Procédé suivant la revendication 2, dans lequel le rhodium est choisi dans le groupe comprenant le rhodium métallique, les oxydes de rhodium, RhI₃, RhBr₃, RhCl₃, Rh(Acac)₃, Rh(CO)₂Acac, [RhCl(CO)₂]₂ et Rh(NO₃)₃.

7. Procédé suivant la revendication 6, dans lequel le rhodium est présent à une concentration de 0,005 à 0,10 M.

8. Procédé suivant la revendication 7, dans lequel la phosphine est une trialkylphosphine.

9. Procédé suivant la revendication 8, dans lequel la phosphine est choisie dans le groupe comprenant la tricyclohexylphosphine, la triisopropylphosphine, la triisobutylphosphine, la tri-n-butylphosphine et la tri-n-propylphosphine.

10. Procédé suivant la revendication 9, dans lequel le rapport molaire du rhodium à la phosphine va de 1:2 à 2:1.

11. Procédé suivant la revendication 10, dans lequel le rapport molaire de l'acide à la phosphine va de 5:1 à 1:5.

12. Procédé suivant la revendication 11, dans lequel le rapport H₂:CO va de 5:1 à 1:1.

13. Procédé suivant la revendication 1, dans lequel le 1,3-glycol est le 1,3-propanediol, l'époxyde est l'oxyde d'éthylène, le solvant est le tétraglyme, la température de réaction va de 100 à 130°C et la pression manométrique de réaction va de 6 996 000 à 20 786 000 Pa (1000 à 3000 lb/in²).
